# EUROPEAN PATENT APPLICATION

(11) **EP 4 480 449 A1**
(43) Date of publication of application: **25.12.2024**
(21) Application number: 23756357.2
(22) Date of filing: 14.02.2023
(51) Int. Cl.: A61D 13/00, A01K 29/00, G01K 13/20

(54) **VETERINARY THERMOMETER AND HEALTH MANAGEMENT SYSTEM USING SAME**

(30) Priority: 17.02.2022 JP 2022023208
(71) Applicant: Higashi, Hayato, Nishinomiya-shi, Hyogo 662-0916 (JP)
(72) Inventor: HIGASHI, Ryuma, Miyakonojo-shi, Miyazaki 885-0224 (JP)
(74) Representative: WSL Patentanwälte Partnerschaft mbB
(86) International application number: PCT/JP2023/004968
(87) International publication number: WO 2023/157832

(57) **Abstract**

The present invention provides: a veterinary thermometer capable of constantly detecting a core temperature of an animal without affecting behaviors of the animal; and a health management system with the same. The veterinary thermometer is suspended from a back of a head of the animal and includes a detector 23 of a temperature instrument 20 which is arranged on an inner cheek portion of the animal.

## Description

### Technical Field

The present invention relates to a veterinary thermometer and a health management system with the same for performing health management for animals such as livestock and pets.

### Background Art

Conventionally, in the health management for animals such as livestock and pets, body temperature is an important indicator of health conditions, and core body temperature should be measured to check the accurate body temperature. The measurement of the core body temperature of animals is generally very laborious and stressful for not only measurers but also the animals because the animals should be held and kept in a fixed position with a thermometer inserted into the rectum until the measurement is completed.

In livestock industries, a large number of livestock such as cows is raised and managed. It takes a lot of time and effort to measure the body temperature of individual livestock for health management. There are not a few cases where animals die due to delayed detection of diseases or other abnormal conditions. Accordingly, for example, a technology has been developed that takes thermal images with a thermographic camera aimed at the head of a calf feeding from a feeding portion, extracts associated temperature from a portion of the head of the calf such as the eyeballs, the area around the eyes, and the inside of the ears, which is considered to be highly associated with core body temperature, and manages the health conditions based on the associated temperature (see Patent Literature 1).

### Citation List

### Patent Literature

Patent Literature 1: Japanese Patent Application Publication No. 2019-58079 (Pages 4-6, FIG.1)

### Summary of the Invention

### Technical Problem

In the technology described in Patent Literature 1, the thermography camera can be used to measure the body temperature of animals in a non-contact manner without affecting the behaviors of the animals such as feeding or drinking, and can significantly reduce the labor and stress of the measurer and the animals. However, the technology in Patent Literature 1 can measure the body temperature of animals only at a timing of a specific behavior such as feeding or drinking, which may overlook a change in body temperature due to a sudden deterioration in health of, for example, a calf or adult cattle before or after giving birth, resulting in late detection of abnormal conditions.

The present invention has been made to solve these problems, and aims to provide a veterinary thermometer and a health management system with the same that can constantly detect deep temperature without affecting behaviors of animals.

### Means for Solving the Problem

In order to the solve the above problems, a veterinary thermometer according to the present invention is a veterinary thermometer configured to be suspended from a back of a head of an animal and including a detector of a temperature instrument, wherein the detector is configured to be arranged on an inner cheek portion. According to the aforesaid feature of the present invention, since the detector of the temperature instrument is arranged on the inner cheek portion of the animal with the veterinary thermometer suspended from the back of the head of the animal, an oral temperature, that is, a core body temperature of the animal can be constantly detected without affecting the behaviors of the animal such as feeding, drinking, and browsing.

It may be preferable that the veterinary thermometer further includes an engaging portion configured to be engaged with a mouth corner of the animal. According to this preferable configuration, since the engaging portion is engaged with the mouth corner of the animal, the veterinary thermometer is easily brought in close contact with the head of the animal. Accordingly, the detector of the temperature instrument arranged on the inner cheek portion of the animal can be less likely to be displaced to stably detect the oral temperature of the animal.

It may be preferable that the veterinary thermometer further includes a frame configured to extend around the back of the head of the animal, and the detector is provided in the engaging portion formed in the frame. According to this preferable configuration, only the frame can be used to stably arrange the detector of the temperature instrument on the inner cheek portion of the animal.

It may be preferable that the veterinary thermometer further includes a restricting portion configured to surround a nasal bone portion and a lower jaw of the animal. According to this preferable configuration, since the restricting portion surrounds the outer periphery of the nasal bone portion and the lower jaw of the animal, the head of the animal can be sandwiched between the restricting portion and a portion of the veterinary thermometer suspended from the back of the head of the animal to securely fix the veterinary thermometer to the head of the animal, preventing the veterinary thermometer from being displaced and falling off from the head.

It may be preferable that at least the detector is covered with a metal cover. According to this preferable configuration, the metal cover protects the detector of the temperature instrument, which is arranged on the inner cheek portion of the animal, such that the damage of the detector due to the bite by the animal can be prevented.

It may be preferable that the veterinary thermometer further includes an auxiliary fixing portion configured to extend along a lower side of the lower jaw of the animal, and the auxiliary fixing portion is provided with a processing portion of the temperature instrument. According to this preferable configuration, since the processing portion of the heavy temperature instrument is supported on the lower side of the lower jaw, which is less affected by the swinging motion of the animal, centrifugal force is less likely to act on the processing portion, preventing the displacement of the detector.

A health management system according to the present invention is a health management system is configured for reporting an abnormal condition to a management terminal based on difference between an oral temperature of the animal detected by the veterinary thermometer and a preset reference temperature. According to the aforesaid feature of the present invention, the system can constantly detect the core body temperature of the animal to which the veterinary thermometer is attached and report abnormal conditions to the management terminal without overlooking any changes in core body temperature of the animal due to poor physical conditions, enabling early detection of diseases while reducing the effort involved in health management of the animal.

### Brief Description of Drawings

FIG. 1 is a perspective view showing a veterinary thermometer according to a first embodiment of the present invention.
FIG. 2 is a plan view of the veterinary thermometer according to the first embodiment.
FIG. 3 is a partial cross-sectional view illustrating hook-shaped portions of a frame attached to opposite mouth corners of a cow. FIG. 3 shows a lower jaw of the cow as viewed from above.
FIG. 4 is a side view showing the veterinary thermometer according to the first embodiment attached to a cow.
FIG. 5 is a front view showing the veterinary thermometer according to the first embodiment attached to a cow.
FIG. 6 is an enlarged view of a main portion showing a metal cover attached to the frame of the veterinary thermometer according to the first embodiment.
FIG. 7 is a perspective view showing the veterinary thermometer according to the first embodiment with an auxiliary fixture attached.
FIG. 8 is a side view showing the veterinary thermometer of FIG. 7 attached to a cow.
FIG. 9 is a view showing an overview of a health management system with a veterinary thermometer.
FIG. 10 is a side view showing a veterinary thermometer according to a second embodiment of the present invention attached to a cow.
FIG. 11 is a front view showing the veterinary thermometer according to the second embodiment attached to a cow.
FIG. 12(a) is a front view showing a veterinary thermometer according to a third embodiment of the present invention, and FIG. 12(b) is a rear view of the same.
FIG. 13 is a side view showing the veterinary thermometer according to the third embodiment.
FIG. 14 is a perspective view showing a veterinary thermometer according to a fourth embodiment of the present invention.
FIG. 15 is a perspective view showing a modification of the veterinary thermometer according to the fourth embodiment.
FIG. 16 is a perspective view showing a veterinary thermometer according to a fifth embodiment of the present invention.
FIG. 17(a) and (b) are views showing the state in which the length of a base portion of a frame has been adjusted with a tube connector.
FIG. 18 is a side view showing the veterinary thermometer in FIG. 16 attached to a cow.

### Modes for Carrying out the Invention

Modes for implementing a veterinary thermometer and a health management system with the same according to the invention are described below according to embodiments.

### First Embodiment

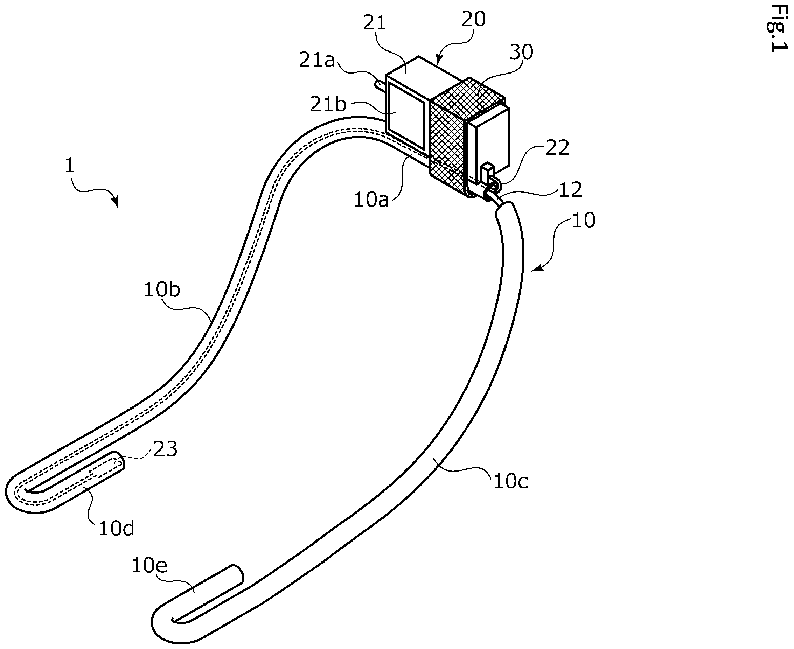

A veterinary thermometer and a health management system with the same according to a first embodiment of the present invention will be described with reference to FIGS. 1 to 9. In the description below, the left side of the page in FIG. 1 will be referred to as the front (front side) of the veterinary thermometer, and the right side of the page will be referred to as the rear (back side) of the veterinary thermometer.

The veterinary thermometer of the present invention is applied to an animal such as livestock and pets. The thermometer is suspended from a back of a head of an animal and includes a detector of a temperature instrument which is arranged on an inner cheek portion of the animal. The detector constantly detects an oral temperature highly associated with the core body temperature of the animal. In this embodiment, a case will be illustrated in which the veterinary thermometer is applied to a cow raised and managed as livestock.

It should be noted that being suspended means being attached in a hanging state. In this embodiment, it means being attached with a portion of the veterinary thermometer supported at the back of the head of the animal and the other portions of the veterinary thermometer hanging down.

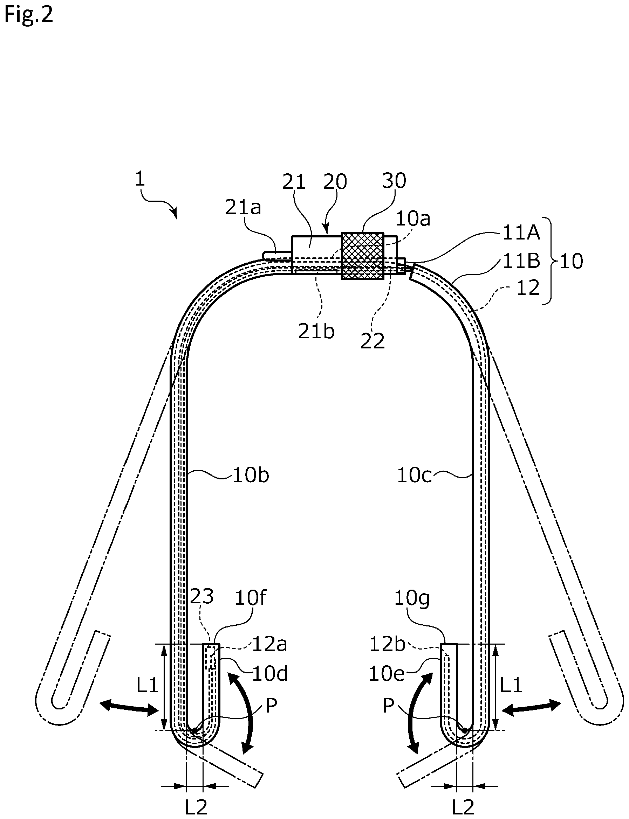

As shown in FIG. 1, a veterinary thermometer 1 (hereinafter referred to as "thermometer 1") mainly includes a frame 10 curved into a substantially reverse U-shape, and a temperature instrument 20 fixed to the frame 10. FIGS. 1 and 2 illustrate the profile of the frame 10 with the thermometer 1 attached to the head of the cow in the first embodiment.

As shown in FIGS. 1 and 2, the frame 10 includes a base portion 10a to which a temperature instrument body 21 as a processing portion of the temperature instrument 20, which will be described later, is fixed, a pair of right and left arm portions 10b, 10c extending from the opposite sides of the base portions 10a, and hook-shaped portions 10d, 10e as engaging portions formed by bending the end portions of the arm portions 10b, 10c inward at an angle of approximately 180 degrees, respectively. The frame 10 is formed such that it has a total length of approximately 700 to 800 mm for a calf, and approximately 1400 to 1600 mm for adult cattle.

As shown in FIG. 2, in this embodiment, the hook-shaped portions 10d, 10e are formed in a symmetrical shape such that they have a dimension L1 of approximately 40 to 60 mm from a point P radially inward of the approximately semicircular bent portion to opposite ends 10f, 10g of the frame 10. The hook-shaped portions 10d, 10e are also formed such that they have a width L2 of the clearance portion of approximately 7 to 10 mm for a calf, and approximately 10 to 20 mm for adult cattle.

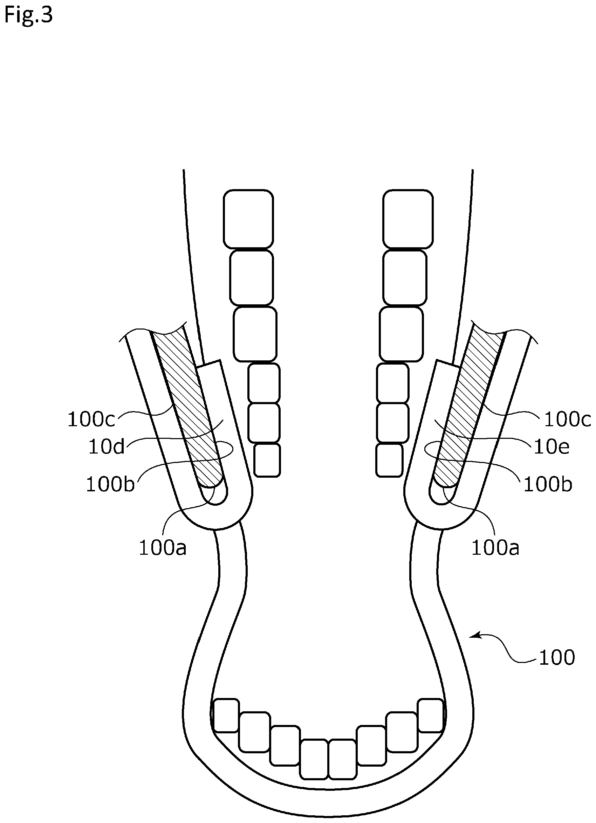

Accordingly, when the hook-shaped portions 10d, 10e are engaged with opposite mouth corners 100a of a cow 100, each of the hook-shaped portions 10d, 10e can be brought into close contact along with inner cheek portions 100b and outer cheek portions 100c of the cow 100 (see FIGS. 3 to 5) .

The mouth corners are the portions on opposite sides of the mouth of an animal where the upper and lower lips connect. The inner cheek portions are portions of cheek portions on the inside of the mouth between the upper and lower jaws of an animal, and the outer cheek portions are portions of the cheek portions on the outside of the mouth.

As shown in FIG. 2, the frame 10 in the first embodiment includes polyurethane tubes 11A, 11B and a stainless steel core 12 extending through the tubes 11A, 11B, and the entire frame 10 is plastically deformable.

In this embodiment, the tubes 11A, 11B are circular tubes each having an outer diameter of about 5 to 6 mm and an inner diameter of about 3 to 4 mm. The core 12 is a wire rod with a circular cross section and a diameter of about 2 to 3 mm. Preferably, a clearance portion of approximately 1 to 2 mm may be formed between the inner peripheral surfaces of the tubes and the outer peripheral surface of the core, through which a lead wire 22 and a detector 23 of the temperature instrument 20, which will be described later, pass.

The core 12 also has opposite ends 12a, 12b of the core 12 located deeper into the interior than the ends of the tubes 11A, 11B, that is, the opposite ends 10f, 10g of the frame 10.

As shown in FIGS. 1 and 2, the temperature instrument 20 in the first embodiment is a so-called digital temperature instrument, and includes: the temperature instrument body 21 in which a battery, a wireless module, or the like (not shown) is built in; the lead wire 22 extending from the temperature instrument body 21 and extending through the inside of the tube 11A constituting the arm portion 10b and the hook-shaped portion 10d of the frame 10; and the detector 23 provided at the end of the lead wire 22 and built in the side of the end 10f of the hook-shaped portion 10d.

The temperature instrument 20 in the first embodiment can convert the temperature detected by the detector 23 into a signal at the temperature instrument body 21, and transmit the signal via an antenna 21a to a management terminal constituting a health management system, which will be described later.

A fixing tape 30 is used to integrally fix the temperature instrument body 21 to a portion of the frame 10 that extends behind the head of the cow 100, that is, the base portion 10a. Fixing means other than the fixing tape 30 may be used to fix the temperature instrument body 21 to the base portion 10a of the frame 10.

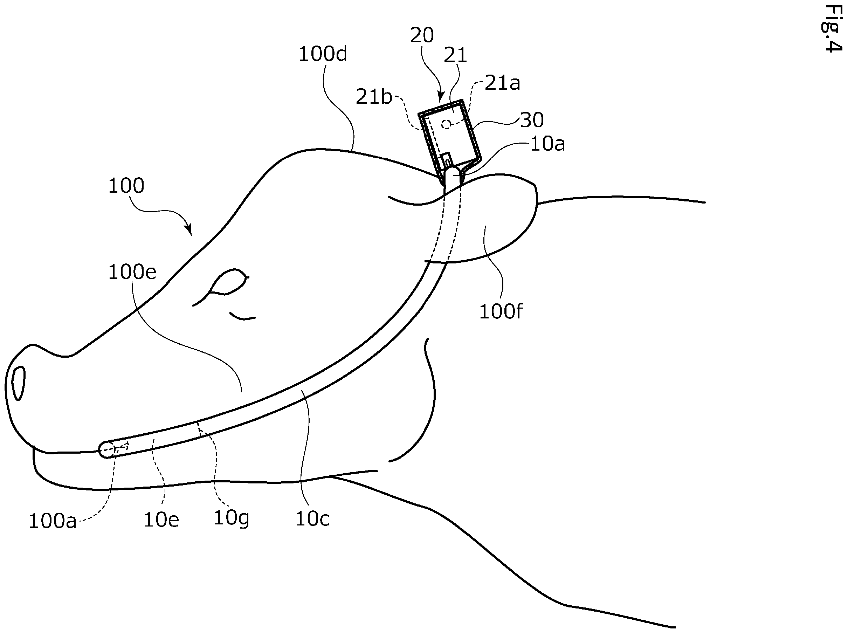

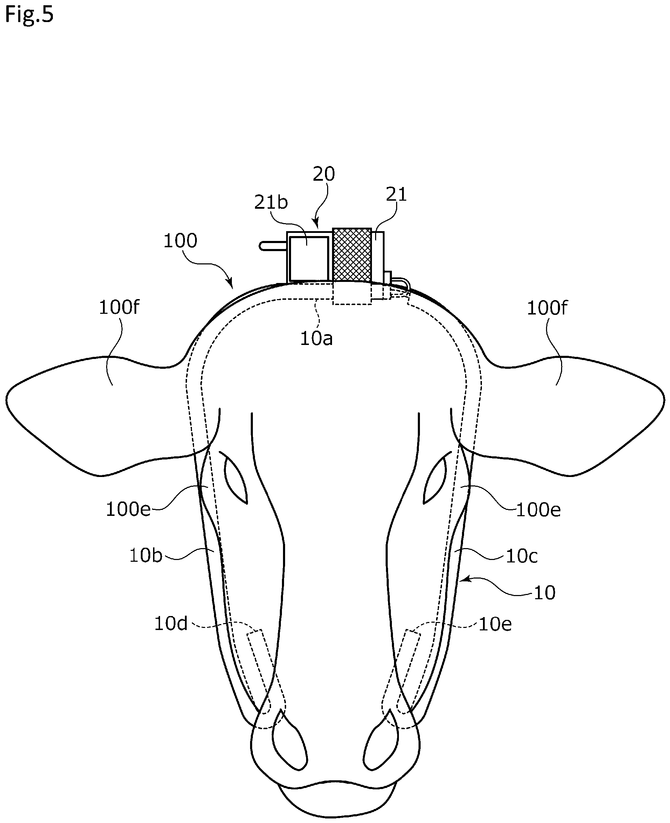

The temperature instrument body 21 is provided with a display screen 21b on which various information such as the temperature detected by the detector 23 and the remaining battery level is displayed. The display screen 21b is arranged to face the front with the frame 10 attached to the head of the cow 100 (see FIGS. 4 and 5).

In this embodiment, the detector 23 built into the hook-shaped portion 10d is a contact temperature sensor for detecting the oral temperature of the cow 100. The detector is not limited to a contact temperature sensor, but may be a non-contact temperature sensor such as an infrared temperature sensor. When the detector is constituted by an infrared temperature sensor, for example, the amount of infrared rays emitted from the inner cheek portion 100b of the cow 100 or the inside of the oral cavity such as the tongue or throat may be measured by the detection surface peeking through the opening of the tube 11A at the end 10f of the frame 10, and the measured amount of infrared rays may be converted into a temperature, such that an oral temperature may be obtained.

Here, an example of a procedure for attaching the thermometer 1 to the cow 100 will be described. The frame 10 will be described on the assumption that the hook-shaped portions 10d, 10e are bent in advance.

First, the thermometer 1 is hung around the neck of the cow 100 with the arm portions 10b, 10c of the frame 10 spread laterally (see FIG. 2). Next, the hook-shaped portions 10d, 10e are engaged with the respective mouth corners 100a of the cow 100 (see FIG. 3). Then, the arm portions 10b, 10c are bent such that they stand behind ears 100f of the cow 100, and the base portion 10a and the temperature instrument body 21 are brought into contact with the vicinity of an occipital region 100d of the cow 100, such that the thermometer is suspended (see FIG. 4) . Finally, the respective arm portions 10b, 10c are curved along the lower side of right and left cheekbone portions 100e of the cow 100 (see FIGS. 4 and 5).

Accordingly, the thermometer 1 of the present invention is suspended from the back of the head (occipital region 100d) of the cow 100 with the frame 10, and the detector 23 of the temperature instrument 20 is arranged on the inner cheek portion 100b of the cow 100, such that it can constantly detect the oral temperature, that is, the core body temperature of the cow 100 without affecting behaviors of the cow 100 such as feeding, drinking, browsing, or ruminating.

Additionally, since the thermometer 1 has the hook-shaped portions 10d, 10e which are engaged with the opposite mouth corners 100a of the cow 100, the thermometer 1 can be reliably brought into close contact with the head of the cow 100 to prevent the displacement of the detector 23 arranged on the inner cheek portion 100b of the cow 100. In addition, since the thermometer 1 is closely attached to the head of the cow 100, the head of the cow 100 and the thermometer 1 can move together to prevent the thermometer 1 from being displaced or falling off from the head.

In addition, since the thermometer 1 includes the frame 10 that extends around the back of the head of the cow 100 to connect the engaging portions, that is, the hook-shaped portions 10d, 10e, and the hook-shaped portion 10d is provided with the detector 23 of the temperature instrument 20. Accordingly, only the frame 10, which is attached to the head of the cow 100 and has the hook-shaped portions 10d, 10e integrally formed therein, can be used to stably arrange the detector 23 on the inner cheek portion 100b of the cow 100.

In addition, since in the thermometer 1, the heavy temperature instrument body 21 is supported at and suspended from the back the head of the cow 100, specifically, in the vicinity of the occipital region 100d, the load from the thermometer 1 can be mostly applied to the vicinity of the occipital region 100d of the cow 100, such that force is less likely to act on the hook-shaped portions 10d, 10e which are engaged with the opposite mouth corners 100a of the cow 100 below the temperature instrument body 21. Accordingly, the hook-shaped portions 10d, 10e can be prevented from being deformed with the frame 10 attached to the head of the cow 100. In other words, force can be less likely to act on the detector 23 built in the hook-shaped portion 10d, which is arranged on the inner cheek portion 100b of the cow 100, to prevent displacement of the detector 23 and stably detect the oral temperature of the cow 100.

Additionally, since the hook-shaped portions 10d, 10e are engaged with the opposite mouth corners 100a of the cow 100 with the base portion 10a of the frame 10 and the temperature instrument body 21 brought into contact with, supported and suspended in the vicinity of the occipital region 100d of the cow 100, attachment of the thermometer 1 to the head of the cow 100 can be maintained in a well-balanced manner. Accordingly, the bent portions of the hook-shaped portions 10d, 10e are prevented from lifting the mouth corners 100a of the cow 100, and the opposite ends 10f, 10g of the frame 10 are prevented from digging into the inner cheek portions 100b of the cow 100, such that the cow 100 with the thermometer 1 attached to the head is less likely to be stressed.

It should be noted that the frame 10 may be formed to have a total length increased by approximately 100 mm such that the arm portions 10b, 10c are curved downward to a greater extent on the lower side of the right and left cheekbone portions 100e of the cow 100 than in the attachment state of the frame 10 shown in FIGS. 4 and 5. Accordingly, as the head of the cow 100 grows, the side of the base portion 10a supported in the vicinity of the occipital region 100d is gradually pulled up with the hook-shaped portions 10d, 10e engaged with opposite mouth corners 100a, such that the curvature of the arm portions 10b, 10c is reduced, that is, the arm portions 10b, 10c are deformed to elongate. Accordingly, the attachment of the frame 10 can be automatically adjusted in accordance with the growth of the head of the cow 100 to reduce the labor involved in adjusting the attachment of the frame 10.

In addition, since the hook-shaped portions 10d, 10e are formed such that the dimension L1 from the point P radially inward of the bent portion to the respective ends 10f, 10g of the frame 10 is approximately 40 to 60 mm (see FIG. 2), the ends 10f, 10g of the frame 10 are less likely to damage the inside of the mouth of the cow 100 as well as can detect the oral temperature of the cow 100 with high accuracy.

Since the hook-shaped portions 10d, 10e are also formed such that the width L2 of the clearance portion is approximately 7 to 10 mm for a calf, and approximately 10 to 20 mm for adult cattle (see FIG. 2), when the hook-shaped portions 10d, 10e are engaged with the respective mouth corners 100a of the cow 100, each of the hook-shaped portions 10d, 10e can be brought into close contact along with the inner cheek portion 100b and the outer cheek portion 100c of the cow 100. Accordingly, the engagement of the hook-shaped portions 10d, 10e can be stably maintained. In addition, the ends 10f, 10g of the hook-shaped portions 10d, 10e are less likely to be bitten by the back teeth called premolars and back molars (see FIG. 3) of the cow 100, and deformation and wearing of the hook-shaped portions 10d, 10e, and damage to the detector 23 built in the hook-shaped portion 10d can be prevented.

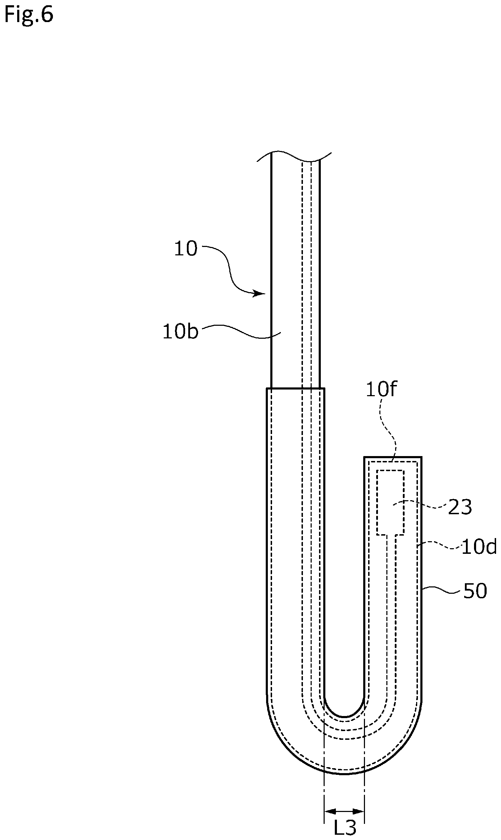

As shown in FIG. 6, the thermometer 1 may include metal covers 50 that protect the hook-shaped portions 10d, 10e of the frame 10. For convenience of explanation, FIG. 6 shows only one of the metal covers 50 that covers the hook-shaped portion 10d, which is a portion of the frame 10 where the detector 23 is disposed.

The metal covers 50 in this embodiment are each formed by a coil portion of a so-called cylindrical tension coil spring made of metal such as stainless steel or piano wire, and the inner diameter thereof is substantially the same as outer diameter of the tubes 11A, 11B constituting the frame 10.

In addition, the metal covers 50 are inserted from the ends 10f, 10g of the frame 10 and then bent inwardly at an angle of approximately 180 degrees together with the end portions of the arm portions 10b, 10c, such that the metal covers 50 are bent and deformed along the bending shape of the hook-shaped portions 10d, 10e. Thus, the metal covers 50 can be held so as not to be misaligned with the frame 10 by utilizing the force of returning to the original linear shape. Accordingly, the respective metal covers 50 can cover and protect the hook-shaped portions 10d, 10e of the frame 10 arranged on the inner cheek portions 100b of the cow 100 to prevent the deformation and wearing of the hook-shaped portions 10d, 10e due to the bite by the back teeth of the cow 100 and the damage of the detector 23 built into the hook-shaped portion 10d.

In this embodiment, the metal covers 50 are formed such that they have a total length of approximately 100 to 140 mm, and can thus cover not only the ends 10f, 10g and the bent portions of the hook-shaped portions 10d, 10e but also the arm portions 10b, 10c. Accordingly, the metal covers 50 are securely held so as not to be misaligned with the frame 10, such that the metal covers 50 can be prevented from falling off from the frame 10 and being swallowed accidentally by animals. In addition, when the respective metal covers 50 are attached to the hook-shaped portions 10d, 10e, the metal covers 50 may preferably have a width L3 (see FIG. 6) of the clearance portion of approximately 7 to 10 mm for an calf and approximately 10 to 20 mm for adult cattle.

In addition, since the opposite ends 12a, 12b of the core 12 constituting the frame 10 are located deeper into the interior than the ends of the tubes 11A, 11B, that is, the opposite ends 10f, 10g of the frame 10, the opposite ends 12a, 12b of the core 12 are less likely to protrude from the opposite ends 10f, 10g of the frame 10, and the inner cheek portion 100b of the cow 100 is prevented from being damaged.

In addition, the frame 10 has the hook-shaped portions 10d, 10e bent in advance before being attached to the head of the cow 100. Accordingly, the hook-shaped portions 10d, 10e can be easily engaged with the opposite mouth corners 100a of the cow 100. Additionally, the detector 23 built in the hook-shaped portion 10d can be reliably arranged at the appropriate position for detecting the oral temperature of the cow 100.

Since the bent portions of the hook-shaped portions 10d, 10e are formed in advance, the tubes 11A, 11B and the core 12 are prevented from moving relative to each other due to an operation of deforming the arm portions 10b, 10c when the frame 10 is attached to the head of the cow 100. Accordingly, the opposite ends 12a, 12b of the core 12 are less likely to protrude from the opposite ends 10f, 10g of the frame 10.

In addition, when the frame 10 is attached to the head of the cow 100, the display screen 21b provided in the temperature instrument body 21 is arranged so as to face the front. Accordingly, various information such as the detected temperature and remaining battery level displayed on the display screen 21b can be easily checked from the front of the cow 100.

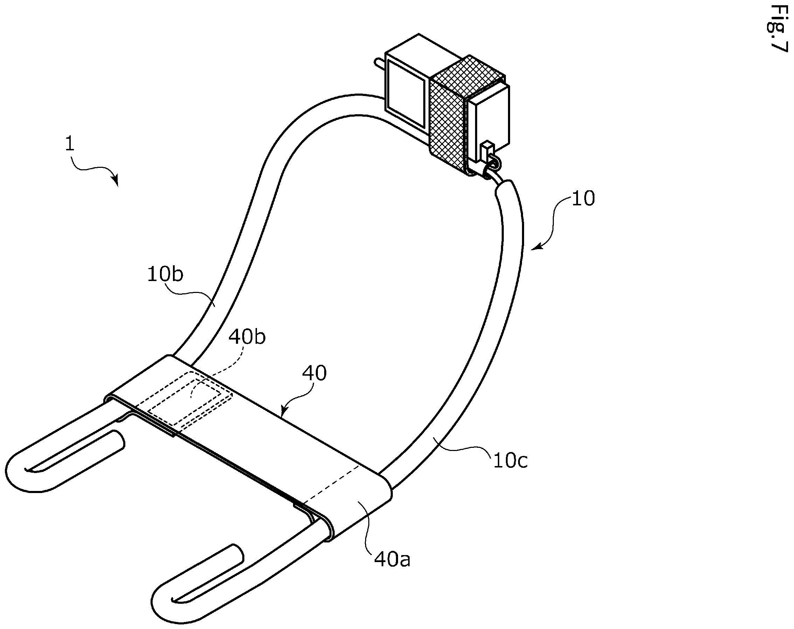

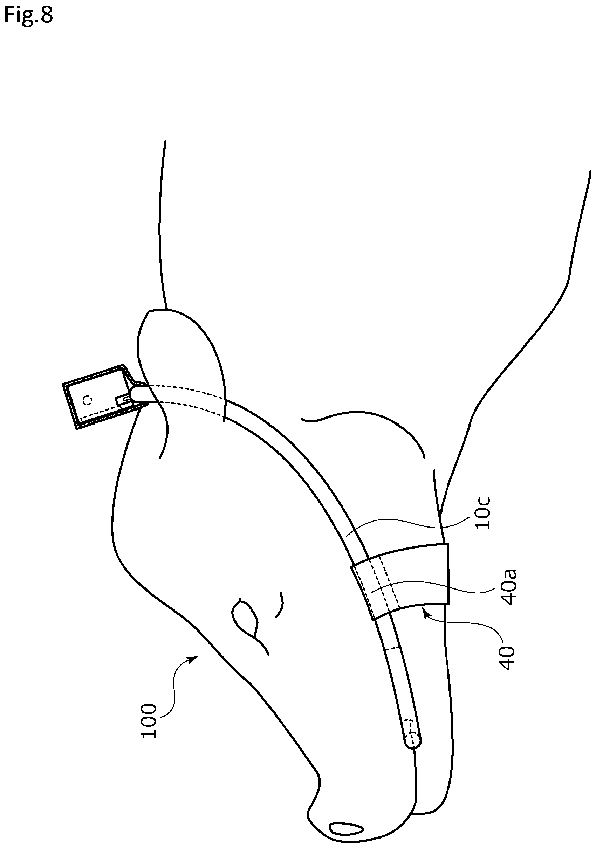

As shown in FIGS. 7 and 8, the thermometer 1 may include a strip-shaped auxiliary fixture 40 connecting the arm portions 10b, 10c of the frame 10.

In this case, as the procedure for attaching the thermometer 1 to the cow 100, with the arm portion 10c being previously inserted through an insertion portion 40a provided at one end of the auxiliary fixture 40, the thermometer 1 is attached to the head of the cow 100 as described above. Then, the strip portion of the auxiliary fixture 40 is placed so as to cover the lower jaw of the cow 100 from below, and the arm portion 10b is fixed by an adhesive portion 40b such as an adhesive seal or a surface seal while being wrapped around the other end of the auxiliary fixture 40. Accordingly, the arm portions 10b, 10c of the frame 10 attached to the head of the cow 100 are connected by the auxiliary fixture 40, and the movement of the thermometer 1 relative to the head of the cow 100 can be further restricted to reliably prevent the thermometer 1 from falling off.

Since the auxiliary fixture 40 is attached so as to extend below the lower jaw of the cow 100, the head of the cow 100, the thermometer 1, and the auxiliary fixture 40 can move together compared to an auxiliary fixture that is attached to the neck of the cow 100, such that the auxiliary fixture 40 is less likely to come off. Additionally, when the thermometer 1 is applied to adult cattle, the auxiliary fixture 40 can be less likely to interfere with a fixture such as a stanchion for tethering the cow to stick the head.

The auxiliary fixture 40 is preferably formed of a suitably stretchable material such as woven fabric, synthetic resin, or rubber. In addition, the shape of the auxiliary fixture 40 is not limited to a strip shape, but may be linear or mesh-like.

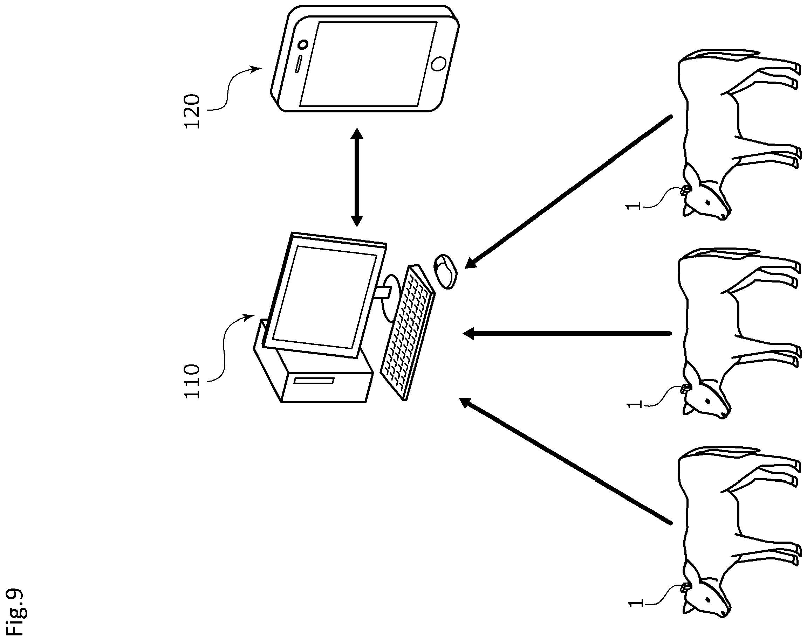

Next, a health management system with the thermometer 1 will be explained with reference to FIG. 9. In this embodiment, a case will be described as an example in which the thermometer 1 is applied to cows kept as livestock, especially calves.

As shown in FIG. 9, control circuits (not shown) of the thermometers 1 attached to a plurality of calves each wirelessly transmits information on the oral temperature detected at preset time intervals (several seconds to several minutes) and the date and time of the detection to a personal computer 110 serving as a management terminal constituting a health management system.

In the health management system, the personal computer 110 manages combined information on the detected temperature and the date and time of the detection received from each of the thermometers 1. Accordingly, the changes in oral temperature can be recorded in time series for each individual to which the thermometer 1 is attached, allowing the health management of the plurality of calves to be performed.

The health management system also reports abnormal conditions from the personal computer 110 to a mobile terminal 120 such as a smartphone or tablet as a management terminal based on the difference between the calf's oral temperature detected by the thermometer 1 and a preset reference temperature. Specifically, the reference temperature is set to 37.5 degrees for a normal temperature of 39.0 degrees for an calf, and when the detected temperature falls below the reference temperature, an alarm or message to call for attention is sent from the personal computer 110 to the mobile terminal 120.

Accordingly, the health management system of the present invention can constantly detect the oral temperature, that is, the core body temperature of the animal to which the thermometer 1 is attached, and can report abnormal conditions to the management terminal without overlooking any changes in core body temperature of the animal due to poor physical conditions, enabling the early detection of disease while reducing the labor involved in health management of the animal.

In addition, since the health management system transmits and receives information wirelessly between the thermometer 1 and the management terminal, it can be applied to animals kept in pasture as well as animals kept in livestock barns. It goes without saying that the health management system can also be applied to animals kept as pets at home, for example.

In the health management system of this embodiment, the preset reference temperature may be set as appropriate depending on the type or growth conditions of animals to which the thermometer 1 is attached. For example, in the case of adult cattle, the reference temperature is set to 37.0 degrees for a normal temperature of 38.5 degrees. Additionally, the difference between the temperature detected by the thermometer 1 and the preset reference temperature, which is a trigger for a report such as an alarm or message to call for attention, may be also set as appropriate depending on the type or growth conditions of animals.

Additionally, in the health management system of this embodiment, an aspect has been described in which the control circuits (not shown) of the thermometers 1 wirelessly transmit information on the detected temperature and the date and time of the detection to the personal computer 110. The present invention is not limited to this, and information on the detected temperature and the date and time of the detection may be transmitted wirelessly from the thermometers 1 directly to the mobile terminal 120 in which an application related to the health management system is installed, and the mobile terminal may be configured to manage combined information on the animal's oral temperature and the date and time of the detection received from the thermometers 1 as well as also report abnormal conditions all at once.

In addition to the information on the animal's oral temperature detected by the thermometer 1, the health management system may also perform overall health management of animals based on various information including raising environment information such as temperature and humidity inside the livestock barns, biological information such as heart rate and respiration rate, and GPS information.

### Second Embodiment

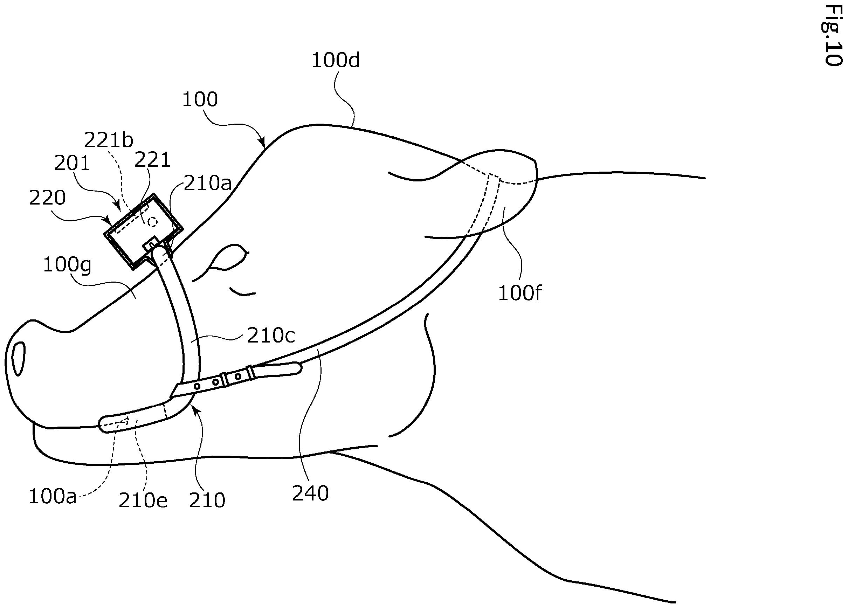

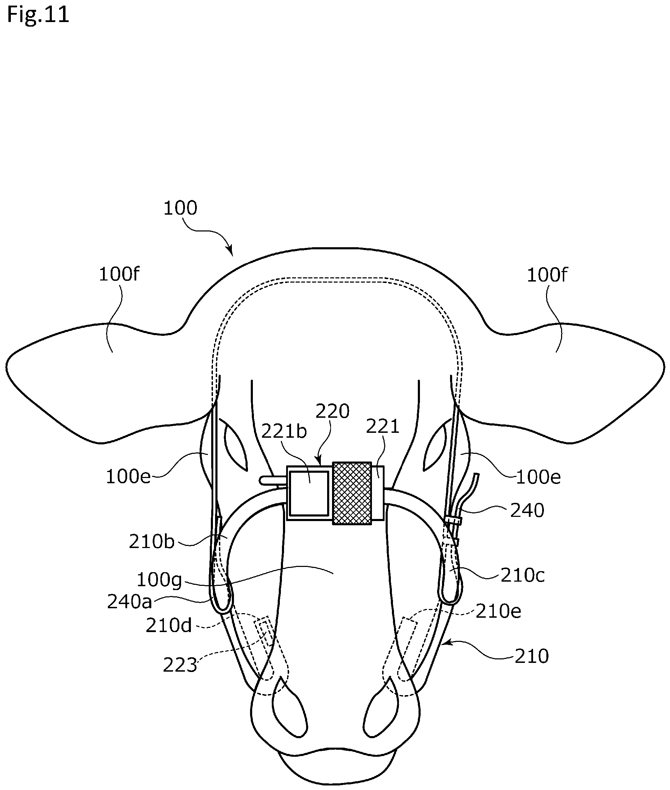

Next, a veterinary thermometer according to a second embodiment of the present invention will be described with reference to FIGS. 10 and 11. Redundant descriptions of the same configurations as those of the first embodiment will be omitted.

As shown in FIGS. 10 and 11, a veterinary thermometer 201 (hereinafter referred to as "thermometer 201") of the second embodiment mainly includes a frame 210, a temperature instrument 220 fixed to the frame 210, and a belt-shaped auxiliary fixture 240 connecting arm portions 210b, 210c of the frame 210. The frame 210 is formed to have a total length of approximately 350 to 400 mm for an calf, and approximately 700 to 800 mm for adult cattle.

Here, an example of a procedure for attaching the thermometer 201 to the cow 100 will be described.

First, with arm portions 210b, 210c of the frame 210 bent at a bending angle of approximately 90 degrees and spread laterally, the thermometer 201 is hung on a nasal bone portion 100g of the cow 100. Next, hook-shaped portions 210d, 210e are engaged with opposite mouth corners 100a of the cow 100, respectively. Then, a ring-shaped insertion portion 240a formed at one end of an auxiliary fixture 240 is inserted into the bent portion of one of the arm portions 210b, and a belt portion of the auxiliary fixture 240 is hung around the neck of the cow 100. The belt portion of the auxiliary fixture 240 is then inserted into the bent portion of the other of the arm portions 210c, and the length of the belt portion is adjusted to fix the auxiliary fixture 240 such that the belt portion is brought into close contact along with the lower side of the cheekbone portion 100e of the cow 100, the back side of the ear 100f, and the vicinity of the occipital region 100d, such that the auxiliary fixture 240 is suspended from the back of the head (occipital region 100d) of the cow 100. Finally, the bending angles of the arm portions 210b, 210c are adjusted to bring a base portion 210a or a temperature instrument body 221 into contact with the nasal bone portion 100g of the cow 100.

Accordingly, the thermometer 201 of the present invention is suspended from the back of the head (occipital region 100d) of the cow 100 with the auxiliary fixture 240, and a detector 223 of the temperature instrument 220 (see FIG. 11) is arranged on the inner cheek portion 100b of the cow 100, such that the oral temperature, that is, the core body temperature of the cow 100 can be constantly detected without affecting the behavior of the cow 100 such as feeding, drinking, browsing, and ruminating. For convenience of explanation, the lead wires of the temperature instrument 220 are not shown in FIG. 11.

In addition, the frame 210 hung on the nasal bone portion 100g of the cow 100 can be anchored by the auxiliary fixture 240 suspended from the back of the head (occipital region 100d) of the cow 100 to bring the thermometer 201 into close contact with the head of the cow 100. Accordingly, the head of the cow 100 and the thermometer 201 can move together to prevent the frame 210 and the auxiliary fixture 240 from being displaced or falling off from the head.

The auxiliary fixture 240 is belt-shaped in the above descriptions, however, the present invention is not limited to this, and may be, for example, string-shaped.

Additionally, the auxiliary fixture 40, which is attached to extend below the lower jaw of the cow 100 described in the first embodiment, may be also applied to the thermometer 201 of the second embodiment, such that the thermometer 201 is ensured to be prevented from falling off from the head of the cow 100.

### Third embodiment

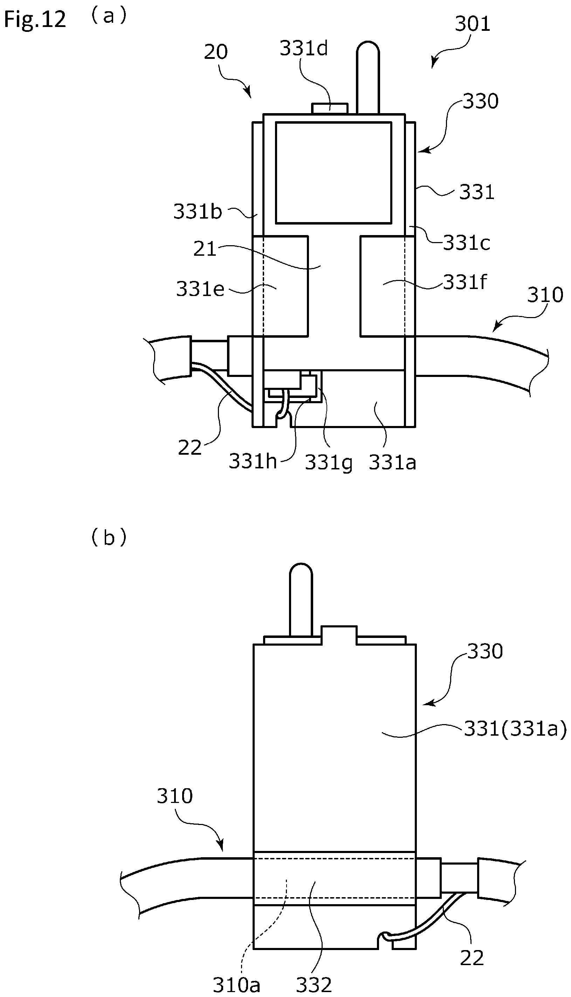

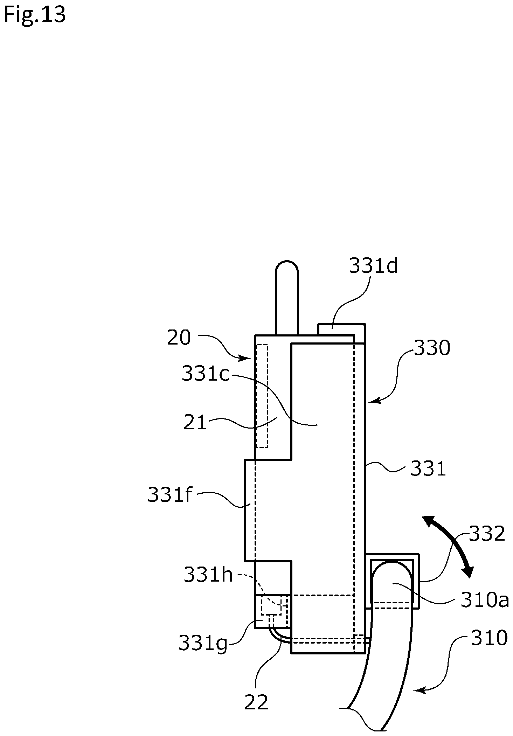

Next, a veterinary thermometer according to a third embodiment of the present invention will be described with reference to FIGS. 12 and 13. Redundant descriptions of the same configurations as those of the first embodiment will be omitted.

As shown in FIGS. 12 and 13, a veterinary thermometer 301 (hereinafter referred to as "thermometer 301") of the third embodiment is different from that of the first embodiment in that the temperature instrument body 21 of the temperature instrument 20 is fixed to a base portion 310a of a frame 310 via a holder 330.

Specifically, the holder 330 is made of a material such as aluminum or other metals or resin, and mainly includes a holding portion 331 capable of holding the temperature instrument body 21 and an insertion portion 332 through which the base portion 310a of the frame 310 is inserted.

The holding portion 331 has a substantially laterally facing U-shape including a back plate 331a and a pair of right and left side plates 331b, 331c standing from opposite sides of the back plate 331a. An L-shaped pressing piece 331d extends upward from the center in the right and left direction of the upper end of the back plate 331a. In addition, L-shaped holding pieces 331e, 331f extend forward from approximately the center in the up and down direction of the front ends of the side plates 331b, 331c so as to face each other. The pressing piece 331d, and the holding pieces 331e, 331f are elastically deformable.

In addition, a rectangular cylindrical protrusion 331g is provided in the lower end portion of the back plate 331a to protrude forward. A notch 331h is formed at the end portion of the protrusion 331g, in which a terminal portion of the lead wire 22 extending from the temperature instrument body 21 is arranged.

When the temperature instrument body 21 is held by the holding portion 331, with the upper portion of the temperature instrument body 21 tilted forward, the temperature instrument body 21 is inserted from the front side of the holding portion 331 and from the upper sides of the holding pieces 331e, 331f. Then, with the bottom portion of the temperature instrument body 21 in contact with the upper surface of the protrusion 331g, the upper portion of the temperature instrument body 21 is rotated rearward, and the pressing piece 331d is then engaged with the back end of the upper portion of the temperature instrument body 21. Accordingly, the holding pieces 331e, 331f and the back plate 331a, the side plates 331b, 331c and the holding pieces 331e, 331f, and the pressing piece 331d and the upper surface of the protrusion 331g restrict the movement of the temperature instrument body 21 in the front and back direction, the movement of the temperature instrument body 21 in the right and left direction, and the movement of the temperature instrument body 21 in the up and down direction, respectively. Accordingly, the temperature instrument body 21 can be protected while being stably held by the holding portion 331.

The insertion portion 332 has a rectangular tube shape and is fixed to the rear side of the holding portion 331 (back plate 331a). The insertion portion 332 is also formed such that its inner peripheral portion is in close contact with the outer peripheral surface of the base portion 310a of the frame 310, and the mounting angle of the holder 330 can be maintained with the base portion 310a of the frame 310 extending through the insertion portion 332. In other words, the mounting angle of the holder 330 can be adjusted by rotating it relative to the base portion 310a of the frame 310 (see the arrow in FIG. 13).

In addition, the shape of the inner peripheral portion of the insertion portion 332 is not limited to the shape described above, and may be formed into a circular shape or a semicircular shape when viewed from the side as long as the holder 330 can exert a holding force to the extent that the holder 330 does not rotate relative to the base portion 310a of the frame 310 due to the weight of the holding portion 331 holding the temperature instrument body 21.

Additionally, with the holding portion 331 holding the temperature instrument body 21, the terminal portion of the lead wire 22 extending from the temperature instrument body 21 can be arranged in the notch 331h of the protrusion 331g, and a part of the lead wire 22 can be routed through the space surrounded by the holding portion 331 to the vicinity of the frame 310, such that the lead wire 22 can be protected.

The present invention is not limited to the holding portion that holds the temperature instrument body with the configuration of the pressing piece, holding piece, and protrusion described above, but the holding portion may be configured to hold the temperature instrument body with a clip, fixing tape, or the like.

### Fourth Embodiment

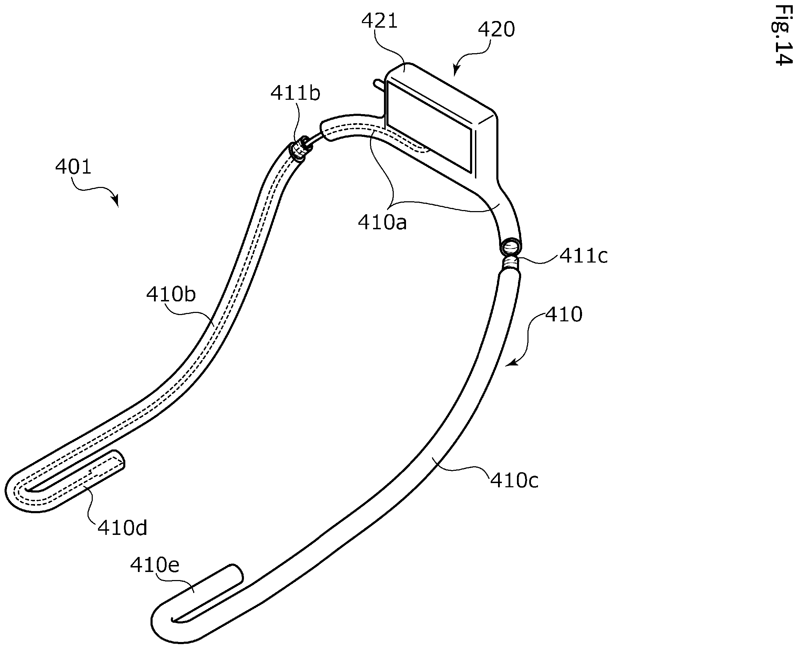

Next, a veterinary thermometer according to a fourth embodiment of the present invention will be described with reference to FIG. 14. Redundant descriptions of the same configurations as those of the first to third embodiments will be omitted.

As shown in FIG. 14, a veterinary thermometer 401 (hereinafter referred to as "thermometer 401") of the fourth embodiment is different from those of the first to third embodiments in that a base portion 410a of a frame 410 is integrally formed with a temperature instrument body 421 of a temperature instrument 420, and arm portions 410b, 410c and hook-shaped portions 410d, 410e are formed separately. It is also different in that the arm portions 410b, 410c and the hook-shaped portions 410d, 410e are formed by tubes made of plastically deformable synthetic resin.

The arm portions 410b, 410c are also provided with connecting portions 411b, 411c having external threads formed on the outer peripheral surfaces at the ends opposite to the hook-shaped portions 410d, 410e. Accordingly, the arm portions 410b, 410c are configured to be detachable such that the connecting portions 411b, 411c are screwed into female threads formed on the inner peripheral surface of the base portion 410a, respectively.

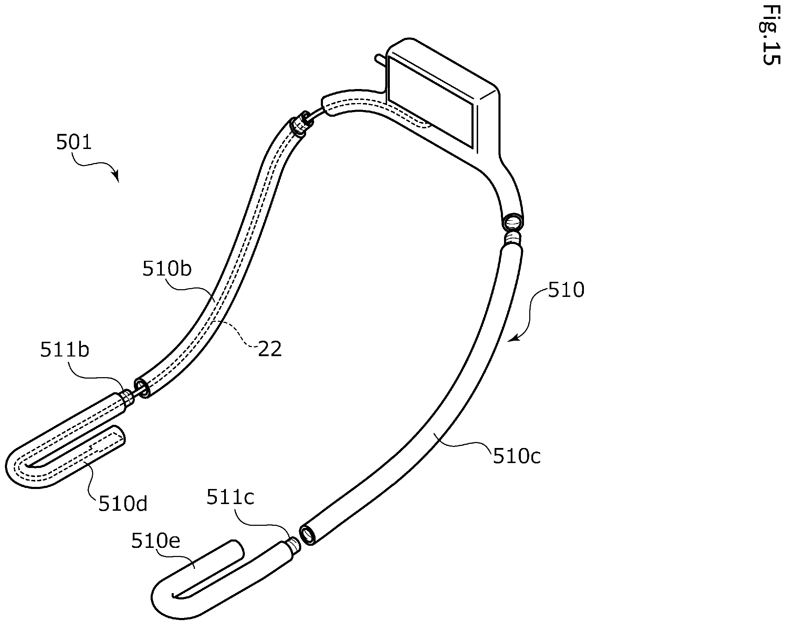

As in a thermometer 501 according to a variation shown in FIG. 15, arm portions 510b, 510c and hook-shaped portions 510d, 510e may be also formed separately, and the connecting portions 511b, 511c having external threads formed on the outer peripheral surface at the ends of the hook-shaped portions 510d, 510e may be provided, such that the arm portions 510b, 510c may be configured to be detachable from the hook-shaped portions 510d, 510e.

Accordingly, an arm portion having an appropriate length can be selectively attached to change the length of the frames 410, 510 according to the size of the head of the animal to which the thermometer 401, 501 is attached.

Additionally, even if the hook-shaped portion is worn out or damaged due to being bitten by an animal, it can be easily replaced.

It should be note that the connecting portions in the arm portions and the hook-shaped portions are not limited to those connecting them with the screw structure as described above, and they may connect them in other connection methods. For example, the frame may have a nested structure, and pins, fixing tape, or the like may be used to connect them such that they do not move relatively.

### Fifth Embodiment

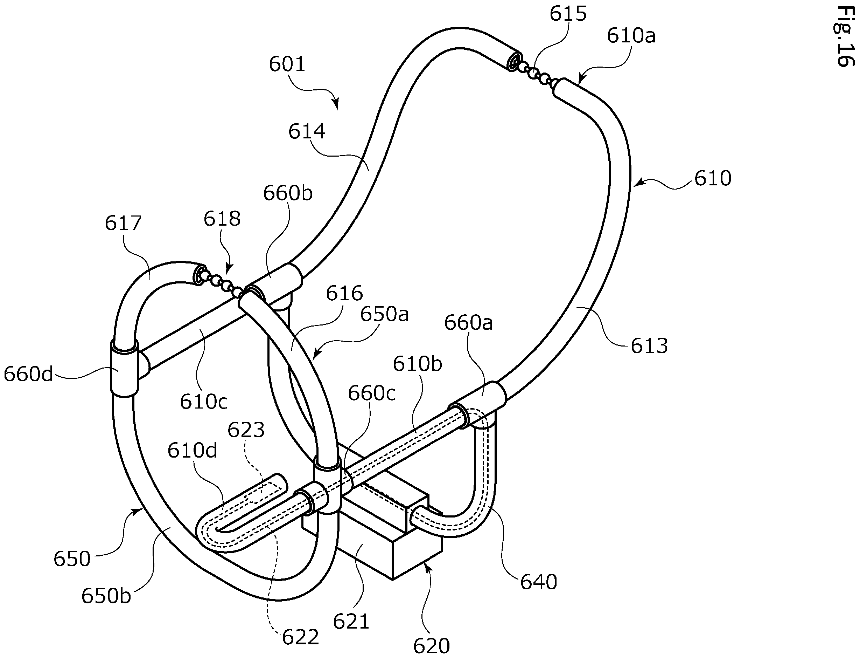

Next, a veterinary thermometer according to a fifth embodiment of the present invention will be described with reference to FIGS. 16 to 18. Redundant descriptions of the same configurations as those of the first to fourth embodiments will be omitted.

As shown in FIG. 16, a veterinary thermometer 601 (hereinafter referred to as "thermometer 601") of the fifth embodiment mainly includes a frame 610, a temperature instrument 620, a temperature instrument installation tool 640 as an auxiliary fixing portion on which the temperature instrument body 621 is installed, and a restrictor 650 as a restricting portion that fixes the frame 610 to the cow 100. It should be noted that the profile of the frame 610 shown in FIG. 16 illustrates the profile when the thermometer 601 is attached to the head of a cow in the fifth embodiment.

The frame 610 mainly includes a base portion 610a which extends around the back of the head of the cow 100, a pair of right and left arm portions 610b, 610c extending from opposite sides of the base portion 610a, and a hook-shaped portion 610d in which a detector 623 is built.

The base portion 610a includes two polyurethane tubes 613, 614 and a tube connector 615 connecting the tubes 613, 614. One ends of the tubes 613, 614 are press-fitted and connected to each other by the tube connector 615, and the other ends of the tubes 613, 614 are detachably connected to adapters 660a, 660b, respectively.

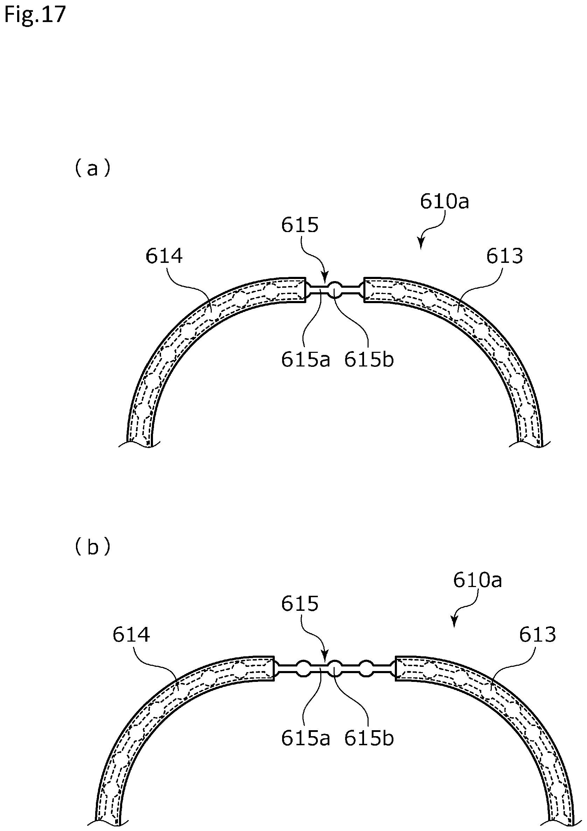

As shown in FIGS. 17(a) and 17(b), the tube connector 615 includes a rod-shaped portion 615a and a plurality of bulging portions 615b having a diameter larger than the rod-shaped portion 615a. The bulging portions 615b are each formed into a spherical shape and are equiangularly arranged in the longitudinal direction of the rod-shaped portion 615a.

The base portion 610a is configured such that the length of the base portion 610a can be changed by changing the press-fit depth of the tube connector 615 into the tubes 613, 614.

Turning to FIG. 16, the arm portions 610b, 610c are formed by polyurethane tubes. One ends of the arm portions 610b, 610c are detachably connected to the adapters 660a, 660b. In other words, the base portion 610a and the arm portions 610b, 610c are connected via the adapters 660a, 660b.

The adapters 660c, 660d are also detachably connected to the other ends of the arm portions 610b, 610c, respectively.

One end of the hook-shaped portion 610d is detachably connected to the adapter 660c on the right side when viewed from the front. The hook-shaped portion 610d is a polyurethane tube with a stainless steel core (not shown) built in. Accordingly, the hook-shaped portion 610d is plastically deformable. The hook-shaped portion 610d is formed such that it first extends from the adapter 660c to the tip side, i.e., the opposite side to the arm portion 610b, and then is bent inward toward the adapter 660c at an angle of approximately 180 degrees.

In this embodiment, an end portion of a core (not shown) built into the hook-shaped portion 610d may extend into the hook-shaped portion 610d in the frame 610 as well as the arm portion 610b, the adapter 660c, and one end side of the temperature instrument installation tool 640 through which the lead wire 622 extending from the temperature instrument body 621 passes. Accordingly, since the core is built into the polyurethane tube, bending deformation of the tube can be prevented to protect the lead wire 622 extending inside the tube from damage.

Additionally, since the core is built in the tube, for example, when one end of the hook-shaped portion 610d and the other end of the arm portion 610b are press-fitted into and connected to the adapter 660c, the tube is prevented from being crushed and deformed in the radial direction. Accordingly, they can be press-fitted and connected more firmly to the adapter 660c than a portion of the tubes in which the core is not built (for example, the base portion 610a of the frame 610, the restrictor 650, and the like). Thus, when the thermometer 601 is attached to the cow 100, the hook-shaped portion 610d, the arm portion 610b, and one end side of the temperature instrument installation tool 640 in the frame 610, in which important parts such as the detector 623 and the lead wire 622 are placed, are difficult to be pulled out from the adapters 660a, 660c.

In this embodiment, a portion of the tubes where the core is not built in is easily elastically deformed, and is easily crushed and deformed in the radial direction compared to a portion of the tubes where the core is built in, making it easier to pull out from the adapters, and to perform maintenance such as adjustment in length or replacement.

In addition, in the frame 610, the hook-shaped portion 610d, the arm portion 610b, and one end side of the temperature instrument installation tool 640, in which important parts such as the detector 623 and the lead wire 622 are placed, may have a different tube color from the other portions such that they are visually clear that there is no need to pull out them from the adapters 660a, 660c during normal use of the thermometer 601.

Additionally, since the core (not shown) extends at least from the hook-shaped portion 610d to the arm portion 610b, even if the hook-shaped portion 610d receives force, for example, due to the movement of the tongue inside the mouth of the cow 100, they are elastically deformable to a certain degree with the hook-shaped portion 610d prevented from rotating relative to the adapter 660c, for example, making it easier for the detector 623 to be arranged on the inner cheek portion while being pressed by the tongue inside the mouth of the cow 100.

The temperature instrument installation tool 640 is a polyurethane tube, and is detachably connected to the adapters 660a, 660b such that it is positioned below the base portion 610a. The temperature instrument 620 is fixed to the temperature instrument installation tool 640 by a fixing means such as a fixing tape. The temperature instrument 620 may be fixed to the temperature instrument installation tool 640 via a holder as described in the third embodiment.

The lead wire 622 extending from the temperature instrument body 621 is connected through the temperature instrument installation tool 640, the adapter 660a, the arm portion 610b, the adapter 660c, and the hook-shaped portion 610d to the detector 623.

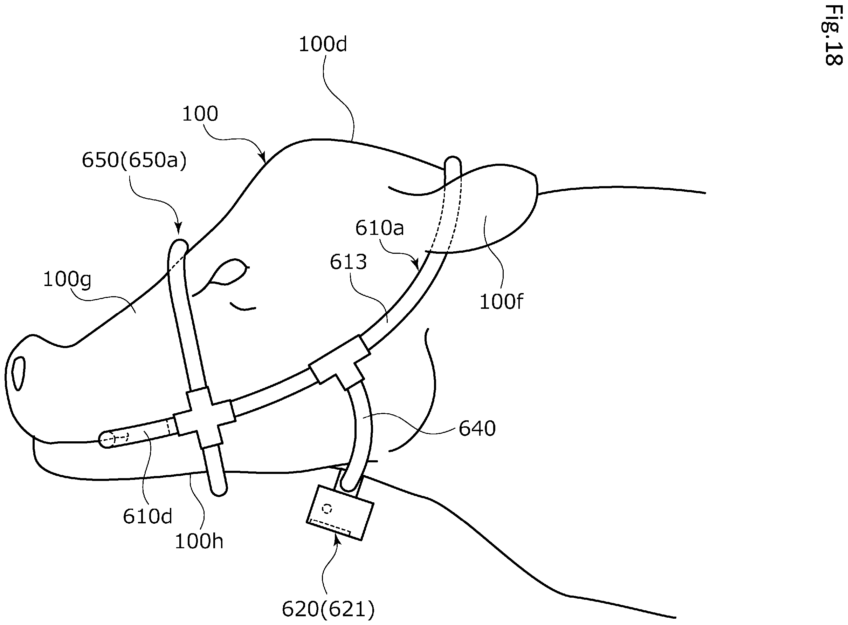

The restrictor 650 is formed into an annular shape. Specifically, the restrictor 650 includes an upper connecting member 650a that is formed into a reverse U shape and connects the adapters 660c, 660d, and a lower connecting member 650b that is formed into a U shape and connects the adapters 660c, 660d. The restrictor 650 is configured to surround the outer periphery of the nasal bone portion 100g and the lower jaw 100h of the cow 100 (see FIG. 18).

The upper connecting member 650a includes two polyurethane tubes 616, 617 and a tube connector 618. The configuration of the upper connecting member 650a is substantially the same as that of the base portion 610a, and a detailed description thereof will be omitted.

Here, an example of a procedure for attaching the thermometer 601 to the cow 100 will be described. It should be noted that the hook-shaped portion 610d is bent in advance.

First, without the tube connector 615 being press-fitted into the tubes 613, 614 of the base portion 610a, the restrictor 650 is inserted from the nose tip side so as to surround the outer periphery of the nasal bone portion 100g and the lower jaw 100h of the cow 100, such that the hook-shaped portion 610d is engaged with the corner of the mouth of the cow 100 (see FIG. 18). Next, the length of the upper connecting member 650a constituting the restrictor 650 is adjusted so as to fit the outer periphery of the nasal bone portion 100g and the lower jaw 100h of the cow 100. Finally, the tube connector 615 is press-fitted into the tubes 613, 614 at the rear side of the ear 100f of the cow 100, and the length of the base portion 610a is adjusted so as to be suspended from the occipital region 100d of the cow 100. Accordingly, the temperature instrument 620 fixed to the temperature instrument installation tool 640 is arranged in the vicinity of the throat at the lower side of the lower jaw 100h of the cow 100.

Accordingly, since the thermometer 601 of the present invention is suspended from the back of the head (occipital region 100d) of the cow 100 and the detector 623 of the temperature instrument 620 is arranged on the inner cheek portion of the cow 100, the oral temperature, i.e., the core body temperature of the cow 100 can be constantly detected without affecting the behaviors of the cow 100 such as feeding, drinking, browsing, and ruminating.

In addition, the head of the cow 100 is sandwiched between the base portion 610a and the restrictor 650 of the frame 610 to restrict the relative movement of the thermometer 601 to the cow 100. Accordingly, the displacement of the detector 623 can be reliably prevented to stably detect the oral temperature of the cow 100.

Additionally, the restrictor 650 is arranged behind the hook-shaped portion 610d, and close to the eyes in the present embodiment not to affect the behaviors of the cow 100, such as feeding, drinking, browsing, and ruminating.

In addition, the base portion 610a of the frame 610 and the restrictor 650 have no built-in core and are easily elastically deformable, preventing digging into the skin, reducing stress on the cow 100, and preventing looseness of attachment to the cow 100.

In accordance with the individual differences and growth of the cow 100, the length of the base portion 610a of the frame 610 and the upper connecting member 650a of the restrictor 650 can be adjusted to properly attach the thermometer 601.

Additionally, the base portion 610a and the upper connecting member 650a are adjusted such that the depth of the tube connector relative to the tube is reduced and the length thereof is longer as the growth of the cow 100 grows. This eliminates the need for a person to adjust the length of the base portion 610a and the upper connecting member 650a in accordance with the growth of the cow 100. In particular, the upper connecting member 650a may produce stress to the cow 100 due to the end of the tube and the tube connector digging into the skin of the nasal bone portion 100g in accordance with the growth of the cow 100. A planar cushioning member may be interposed between the upper connecting member 650a and the nose bone portion 100g to prevent the digging.

In addition, since the temperature instrument body 621 is arranged in the vicinity of the throat that is relatively less likely to move during the swinging motion of the cow 100, the thermometer 601 is prevented from being displaced due to a centrifugal force applied to the temperature instrument body 621 during the swinging motion of the cow 100.

In the fifth embodiment, an aspect in which the length of the base portion 610a and the upper connecting member 650a can be adjusted is described as an example, but the length of the arm portions 610b, 610c, the temperature instrument installation tool 640, the lower connecting member 650b, and the like may be adjusted.

In the fifth embodiment, the hook-shaped portion 610d is provided only on one side, but the hook-shaped portion 610d may be provided on each side.

Additionally, in the fifth embodiment, since the restrictor 650 is provided, the hook-shaped portion 610d as an engaging portion is not necessarily provided as long as the detector 623 faces the inside of the mouth.

Although the embodiments of the present invention have been described above with reference to the drawings, its specific configuration is not limited to these embodiments. Any changes and additions made without departing from the scope of the present invention are included in the present invention.

For example, the first to fourth embodiments describe the frame in which the pair of hook-shaped portions as an engaging portion is engaged with the opposite mouth corners of an animal, but the present invention is not limited to this. As shown in the fifth embodiment, the frame may be engaged with only one of mouth corners of the animal. In this case, for example, the detector of the temperature instrument may be preferably arranged in the hook-shaped portion that is engaged with the mouth corner of the animal.

The second embodiment described above describes the frame that is hooked onto the nasal bone portion of the animal and is anchored by the auxiliary fixture suspended from the back of the head of the animal with the hook-shaped portions engaged with the opposite mouth corners, but the present invention is not limited to this, and the frame may be hooked onto other portions such as the tip of the nose or the lower jaw.

In the embodiments described above, the material of the tubes constituting the frame is not limited to polyurethane, and may be made of other synthetic resins such as silicone or fluororesin, rubber, or metal as long as at least it has flexibility. Additionally, the frame may have no core inserted therein as in the fourth embodiment as long as the tubes themselves are plastically deformable.

Obviously, the thickness of the frame and the dimensional relationship of the hook-shaped portion may be appropriately changed depending on the animals to which the veterinary thermometer of the present invention is applied.

In addition, in the first to third and fifth embodiments, the material of the core is not limited to stainless steel, but may be made of other metals such as aluminum or steel, synthetic resin, or rubber as long as it is plastically deformable.

Additionally, the first to fourth embodiments describe the hook-shaped portions formed symmetrically, but the present invention is not limited to this. For example, the length of the end of the hook-shaped portion placed in the mouth of the animal may vary.

Further, the bent portion of the hook-shaped portion is not limited to being formed in a substantially semicircular shape, and may be bent at an angle of approximately 180 degrees, for example, by being bent at two locations each at approximately 90 degrees.

Further, the frame may be configured such that as long as at least the arm portion of the frame is bendable, the hook-shaped portion is not bendable.

The frame may be also configured to have a variable length. Specifically, for example, the arm portions of the frame may be configured in a nested structure, and a set screw or the like may be used to fix the length of the arm portions during use.

Further, the metal cover described in the first embodiment may also be applied to the veterinary thermometers of the second to fifth embodiments. The metal cover is also not limited to being formed by the coil portion of a cylindrical tension coil spring, and may be formed by other metal coils, metal tubes or rings, or the like as long as it can protect a portion where the detector is located.

Additionally, the frame may be strengthened with a metal tube or the like inserted into a portion of the frame where the detector of the temperature instrument is located, protecting the detector from damage even if an animal bites the end of the hook-shaped portion.

The veterinary thermometer of the present invention may be applied to animals other than cows, such as horses, sheep, goats, and pigs as long as the hook-shaped portion of the frame can be attached to the mouth corner.

The detector in the veterinary thermometer of the present invention may be arranged inside a nostril as long as it can measure body temperature.

### Description of Reference Numeral

1 veterinary thermometer
10 frame
10a base portion
10b, 10c arm portion
10d, 10e hook-shaped portion (engaging portion)
11A, 11B tube (frame)
12 core (frame)
20 temperature instrument
21 temperature instrument body (processing portion)
21a antenna
21b display screen
22 lead wire
23 detector
30 fixing tape
40 auxiliary fixture
50 metal cover
100 cow (animal)
100a mouth corner
100b inner cheek portion
100c outer cheek portion
100d occipital region
100e cheekbone portion
100f ear
100g nasal bone portion
110 personal computer (management terminal)
120 mobile terminal (management terminal)
240 auxiliary fixture
640 temperature instrument installation tool (auxiliary fixing portion)
650 restrictor (restricting portion)

## Claims

1. A veterinary thermometer configured to be suspended from a back of a head of an animal and comprising a detector of a temperature instrument, wherein
the detector is configured to be arranged on an inner cheek portion.

2. The veterinary thermometer according to claim 1, further comprising
an engaging portion configured to be engaged with a mouth corner of the animal.

3. The veterinary thermometer according to claim 2, further comprising
a frame configured to extend around the back of the head of the animal, wherein
the detector is provided in the engaging portion which is formed in the frame.

4. The veterinary thermometer according to claim 1, further comprising
a restricting portion configured to surround a nasal bone portion and a lower jaw of the animal.

5. The veterinary thermometer according to claim 3, wherein
at least the detector is covered with a metal cover.

6. The veterinary thermometer according to claim 1, further comprising
an auxiliary fixing portion configured to extend along a lower side of the lower jaw of the animal, wherein
the auxiliary fixing portion is provided with a processing portion of the temperature instrument.

7. A health management system comprising the veterinary thermometer according to any one of claims 1 to 6, wherein
the health management system is configured for reporting an abnormal condition to a management terminal based on difference between an oral temperature of the animal detected by the veterinary thermometer and a preset reference temperature.
